# EUROPÄISCHE PATENTANMELDUNG

(11) **EP 0 722 944 A1**
(43) Veröffentlichungstag der Anmeldung: **24.07.1996**
(21) Anmeldenummer: 96100156.7
(22) Anmeldetag: 08.01.1996
(51) Int. Cl.: C07D 473/00

(54) **2-9-Disubstituierte Purin-6-one**

(30) Priorität: 19.01.1995 DE 19501482
(71) Anmelder: BAYER AG, 51368 Leverkusen (DE)
(72) Erfinder: Niewöhner, Ulrich, Dr., D-42929 Wermelskirchen (DE); Bischoff, Erwin, Dr., D-42115 Wuppertal (DE); Schütz, Helmuth, Dr., D-42115 Wuppertal (DE); Perzborn, Elisabeth, Dr., D-42327 Wuppertal (DE); Schramm, Matthias, Dr., D-51375 Leverkusen (DE)

(57) **Zusammenfassung**

2,9-Disubstituierte Purin-6-one werden hergestellt, indem man entsprechend substituierte Aminoimidazole in einem ersten Schritt acyliert und danach zum Purin cyclisiert. Die neuen 2,9-disubstituierten Purin-6-one können als Wirkstoffe in Arzneimitteln, insbesondere zur Behandlung von Entzündungen, thromboembolischen, Herz- und Kreislauferkrankungen und Erkrankungen des Urogenitalsystems eingesetzt werden.

## Beschreibung

Die vorliegende Erfindung betrifft 2,9-disubstituierte Purin-6-one, ein Verfahren zu ihrer Herstellung und ihre Verwendung in Arzneimitteln, insbesondere zur Behandlung von Entzündungen, thromboembolischen, Herz- und Kreislauferkrankungen und Erkrankungen des Urogenitalsystems.

Aus den Publikationen J. Pharm. Sci., 74 (10), 1082-85, 1985 und J. Hetero. Chem. 19 (1), 33-40, 1982 sind einige 2-Phenyl-substituierte 6H-Purin-6-one bekannt.

Außerdem sind 1H-9-Methyl-substituierte Purine und Oxazolopyrimidine beschrieben [ vgl. hierzu Khim.-Farm. Zh. 8(4), 26-8, 1974 und Bull. Chem. Soc. Jap. 43 (12), 3509-13 (1970)].

Ferner sind 9-substituierte Hypoxanthine aus JP 47 021 434 bekannt.

Darüber hinaus sind Purinderivate mit einer regulierenden Wirkung auf das Pflanzenwachstum und 2-substituierte-9-(4-methylbenzyl)-9H-purine mit einer Antirhinovirus-Wirkung bekannt [vgl. Egypt. J. Chem., Vol. Data 1990, 33 (3), 243-53, 1991 und J. Med. Chem. 32 (1), 218-24, 1989].

Phosphodiesterasen (PDE's) spielen eine wesentliche Rolle in der Regulation des intrazellulären cGMP und cAMP-Spiegels. Von den bisher beschriebenen Phosphodiesterase-Isoenzymgruppen PDE I bis PDE V [Nomenklatur nach Beavo and Reifsnyder (vgl. Beavo, J.A. and Reifsnyder, D.H.: Trends in Pharmacol. Sci 11, 150 - 155 (1990))] sind die Ca-Calmodulin aktivierte PDE I, die cGMP stimulierbare PDE II und die cGMP spezifische PDE V im wesentlichen für den Metabolismus von cGMP verantwortlich. Aufgrund der unterschiedlichen Verteilung dieser cGMP metabolisierenden PDE's im Gewebe sollten selektive Inhibitoren je nach Gewebsverteilung des entsprechenden Isoenzyms die cGMP-Spiegel im entsprechenden Gewebe anheben. Dieses kann zu einer spezifischen, antiaggregatorischen, antispastischen, gefäßdilatierenden, antiarrhythmischen und/oder antiinflammatorischen Wirkung führen.

Die vorliegende Erfindung betrifft nun 2,9-disubstituierte Purin-6-one der allgemeinen Formel (I), 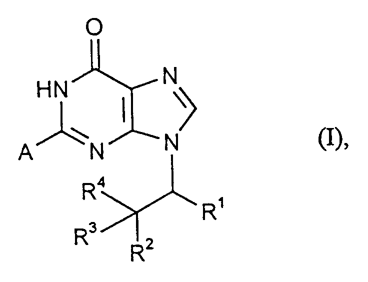
in welcher
- R¹: für geradkettiges oder verzweigtes Alkyl mit 2 bis 10 Kohlenstoffatomen steht, das gegebenenfalls durch Phenyl substituiert ist, das seinerseits durch Halogen, Nitro, Cyano oder durch geradkettiges oder verzweigtes Alkyl mit bis zu 6 Kohlenstoffatomen substituiert sein kann,
- R²: für Wasserstoff, Hydroxy, Azido oder für geradkettiges oder verzweigtes Alkyl mit bis zu 6 Kohlenstoffatomen steht, oder für eine Gruppe der Formel -O-SO₂R⁵ steht,
worin
- R⁵: geradkettiges oder verzweigtes Alkyl mit bis zu 4 Kohlenstoffatomen oder Phenyl bedeutet,
- R³: für Wasserstoff steht,
oder
- R² und R³: gemeinsam den Rest der Formel =O bilden,
- R⁴: für Wasserstoff oder für geradkettiges oder verzweigtes Alkyl mit bis zu 4 Kohlenstoffatomen steht,
und
- A: für einen Rest der Formel 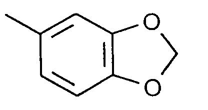 oder
für geradkettiges oder verzweigtes Alkyl mit bis zu 20 Kohlenstoffatomen steht, oder
für Cycloalkyl mit 3 bis 7 Kohlenstoffatomen oder
für Phenyl steht, die gegebenenfalls bis zu 2-fach gleich oder verschieden durch Halogen, Carboxyl, Trifluormethyl, Nitro, Cyano oder durch geradkettiges oder verzweigtes Alkyl, Alkoxycarbonyl oder Alkoxy mit jeweils bis zu 5 Kohlenstoffatomen substituiert sind, die ihrerseits durch Phenyl substituiert sein können, und/oder die Cyclen gegebenenfalls durch Phenyl substituiert sind, das seinerseits durch geradkettiges oder verzweigtes Alkoxy mit bis zu 5 Kohlenstoffatomen substituiert sein kann,
und deren Tautomere und Salze.

Die erfindungsgemäßen Stoffe können auch als Salze vorliegen. Im Rahmen der Erfindung sind physiologisch unbedenkliche Salze bevorzugt.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen der allgemeinen Formel (I) können in verschiedenen stereochemischen Formen auftreten, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Bevorzugt sind Verbindungen der allgemeinen Formel (I),
in welcher
- R¹: für geradkettiges oder verzweigtes Alkyl mit 2 bis 8 Kohlenstoffatomen steht, das gegebenenfalls durch Phenyl substituiert ist, das seinerseits durch Fluor, Chlor, Brom, Nitro, Cyano oder durch geradkettiges oder verzweigtes Alkyl mit bis zu 4 Kohlenstoffatomen substituiert sein kann,
- R²: für Wasserstoff, Hydroxy, Azido oder für geradkettiges oder verzweigtes Alkyl mit bis zu 4 Kohlenstoffatomen oder eine Gruppe der Formel -O-SO₂-R⁵ steht,
worin
- R⁵: geradkettiges oder verzweigtes Alkyl mit bis zu 3 Kohlenstoffatomen oder Phenyl bedeutet,
- R³: für Wasserstoff steht,
oder
- R² und R³: gemeinsam den Rest der Formel =O bilden,
- R⁴: für Wasserstoff oder für geradkettiges oder verzweigtes Alkyl mit bis zu 3 Kohlenstoffatomen steht, und
- A: für einen Rest der Formel 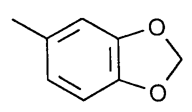 oder
für geradkettiges oder verzweigtes Alkyl mit bis zu 19 Kohlenstoffatomen steht, oder
für Cyclopropyl, Cyclopentyl, Cyclohexyl, Cylcoheptyl oder
für Phenyl steht, die gegebenenfalls bis zu 2-fach gleich oder verschieden durch Fluor, Chlor, Brom, Carboxyl, Nitro, Hydroxy oder durch geradkettiges oder verzweigtes Alkyl, Alkoxycarbonyl oder Alkoxy mit jeweils bis zu 4 Kohlenstoffatomen substituiert sind, die ihrerseits durch Phenyl substituiert sein können, und/oder die Cyclen gegebenenfalls durch Phenyl substituiert sind, das seinerseits durch geradkettiges oder verzweigtes Alkoxy mit bis zu 4 Kohlenstoffatomen substituiert sein kann,
und deren Tautomere und Salze.

Besonders bevorzugt sind Verbindungen der allgemeinen Formel (I),
in welcher
- R¹: für geradkettiges oder verzweigtes Alkyl mit 2 bis 7 Kohlenstoffatomen steht, das gegebenenfalls durch Phenyl substituiert ist, das seinerseits durch Fluor, Chlor, Brom, Nitro, Cyano, durch geradkettiges oder verzweigtes Alkyl mit bis zu 3 Kohlenstoffatomen substituiert sein kann,
- R²: für Wasserstoff, Hydroxy, Azido oder für geradkettiges oder verzweigtes Alkyl mit bis zu 3 Kohlenstoffatomen oder eine Gruppe der Formel - OSO₂R⁵ steht,
worin
- R⁵: geradkettiges oder verzweigtes Alkyl mit bis zu 3 Kohlenstoffatomen oder Phenyl bedeutet,
- R³: für Wasserstoff steht,
oder
- R² und R³: gemeinsam den Rest der Formel =O bilden,
- R⁴: für Wasserstoff oder für geradkettiges oder verzweigtes Alkyl mit bis zu 3 Kohlenstoffatomen steht, und
- A: für einen Rest der Formel 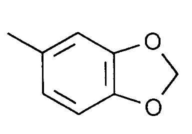 oder
für geradkettiges oder verzweigtes Alkyl mit bis zu 18 Kohlenstoffatomen steht, oder
für Cyclopropyl, Cyclopentyl, Cyclohexyl, Cycloheptyl oder
Phenyl steht, die gegebenenfalls bis zu 2-fach gleich oder verschieden durch Fluor, Chlor, Brom, Carboxyl, Nitro, Hydroxy oder durch geradkettiges oder verzweigtes Alkyl, Alkoxycarbonyl oder Alkoxy mit jeweils bis zu 3 Kohlenstoffatomen substituiert sind, die ihrerseits durch Phenyl substituiert sein können, und/oder die Cyclen gegebenenfalls durch Phenyl substituiert sind, das seinerseits durch geradkettiges oder verzweigtes Alkoxy mit bis zu 3 Kohlenstoffatomen substituiert sein kann,
und deren Tautomere und Salze.

Außerdem wurde ein Verfahren zur Herstellung der erfindungsgemäßen Verbindungen der allgemeinen Formel (I) gefunden, dadurch gekennzeichnet, daß man zunächst Verbindungen der allgemeinen Formel (II) 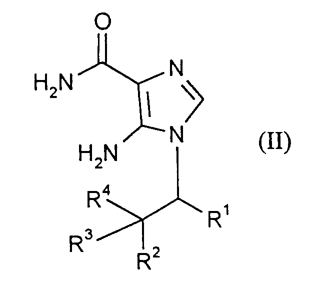
in welcher
R¹, R², R³ und R⁴ die oben angegebenen Bedeutungen haben,
durch Umsetzung mit Verbindungen der allgemeinen Formel (III)

A-CO-Cl (III)

in welcher
- A: die oben angegebene Bedeutung hat,
in inerten Lösemitteln und in Anwesenheit einer Base,
in die Verbindungen der allgemeinen Formel (IV) 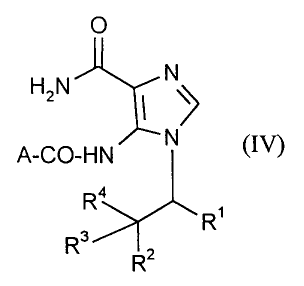
in welcher
A, R¹, R², R³ und R⁴ die oben angegebene Bedeutung haben,
überführt,
in einem zweiten Schritt in inerten Lösemitteln und in Anwesenheit einer Base cyclisiert,
und die Substituenten R¹, R², R³ und R⁴ durch Acylierung, Oxidation und/oder Azidaustausch einführt bzw. derivatisiert.

Das erfindungsgemäße Verfahren kann durch folgendes Formelschema beispielhaft erläutert werden: 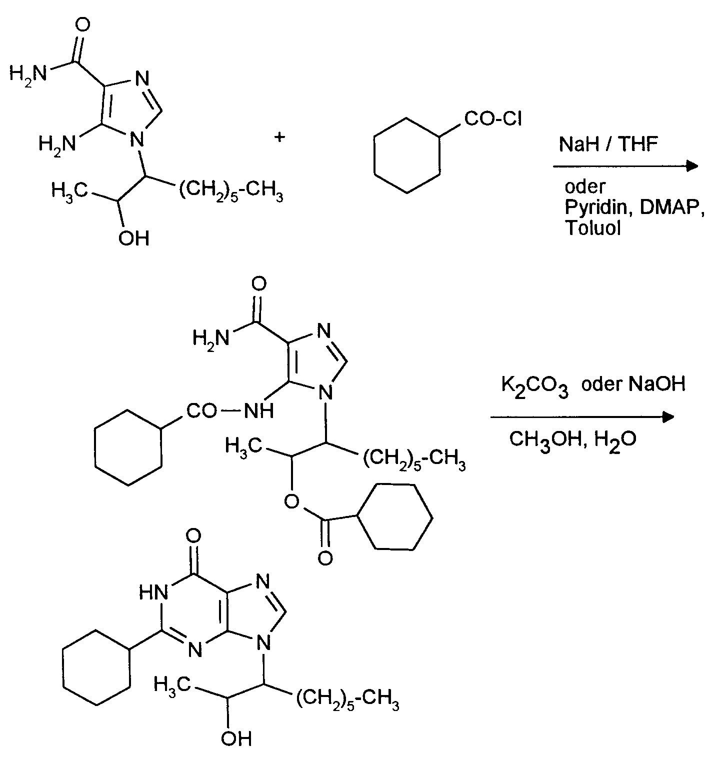
Für den ersten Schritt des Verfahrens eignen sich inerte organische Lösemittel, die sich unter den Reaktionsbedingungen nicht verändern. Hierzu gehören bevorzugt Ether wie beispielsweise Diethylether, Dioxan, Tetrahydrofuran, Glykolmono- oder dimethylether, Halogenkohlenwasserstoffe wie Di-, Tri- oder Tetrachlormethan, Dichlorethylen, Trichlorethylen, Essigester, Toluol, Acetonitril, Hexamethylphosphorsäuretriamid, Pyridin und Aceton. Selbstverständlich ist es möglich, Gemische der Lösemittel einzusetzen. Besonders bevorzugt sind Tetrahydrofuran, Toluol oder Pyridin.

Als Basen eignen sich im allgemeinen Alkalihydride oder -alkoholate, wie beispielsweise Natriumhydrid oder Kalium-tert.butylat, oder cyclische Amine, wie beispielsweise Piperidin, Pyridin, Dimethylaminopyridin oder C₁-C₄-Alkylamine, wie beispielsweise Triethylamin. Bevorzugt sind Natriumhydrid, Pyridin oder Dimethylaminopyridin.

Die Base wird im allgemeinen in einer Menge von 1 mol bis 4 mol, bevorzugt von 1,2 mol bis 3 mol jeweils bezogen auf 1 mol der Verbindungen der allgemeinen Formel (II) eingesetzt.

Die Reaktionstemperatur kann im allgemeinen in einem größeren Bereich variiert werden. Im allgemeinen arbeitet man in einem Bereich von -20°C bis 200°C, bevorzugt von 0°C bis 25°C.

In einer Variante wird die Umsetzung in Pyridin, dem eine katalytische Menge DMAP zugesetzt wird, durchgeführt. Gegebenenfalls kann noch Toluol zugefügt werden.

Als Lösemittel für die Cyclisierung eignen sich die üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Als Basen eignen sich für die Cyclisierung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt sind Kaliumcarbonat, und Natriumhydroxid.

Bei der Durchführung der Cyclisierung wird die Base im allgemeinen in einer Menge von 2 bis 6 mol, bevorzugt von 3 bis 5 mol bezogen auf 1 mol der Verbindungen der Formel (IV), eingesetzt.

Die Cyclisierung wird im allgemeinen in einem Temperaturbereich von 0°C bis 160°C, bevorzugt bei der Siedetemperatur des jeweiligen Lösemittels durchgeführt.

Die Cyclisierung wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Umsetzung mit Alkylsulfonsäurechloriden erfolgt, ausgehend von den entsprechenden freien Hydroxyverbindungen, in einem der oben aufgeführten Lösemittel und einer der Basen, vorzugsweise mit Dichlormethan und Triethylamin in einem Temperaturbereich von -20°C bis +20°C, vorzugsweise 0°C und Normaldruck.

Die Einführung des Azidrestes erfolgt im allgemeinen durch Umsetzung der entsprechenden Alkylsulfonyloxy substituierten Verbindungen mit Natriumazid in einem der oben aufgeführten Lösemittel, vorzugsweise Dimethylformamid, in einem Temperaturbereich von 50°C bis +120°C, vorzugsweise 100°C und Normaldruck.

Die Ketone werden ausgehend von den entsprechenden Hydroxyverbindungen nach bekannten Methoden (Swern-Oxidation) hergestellt.

Die enantiomerenreinen Verbindungen sind nach üblichen Methoden, beispielsweise durch Chromatographie der racemischen Verbindungen der allgemeinen Formel (I) auf chiralen Phasen zugänglich.

Die Verbindungen der allgemeinen Formel (III) sind bekannt.

Die Verbindungen der allgemeinen Formel (IV) sind größtenteils neu und können beispielsweise wie oben beschrieben hergestellt werden.

Die Verbindungen der allgemeinen Formel (II) sind größtenteils neu und können beispielsweise hergestellt werden, indem man
2-Amino-2-cyanoacetamid der Formel (V) 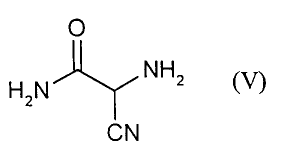
mit Verbindungen der allgemeinen Formel (VI) 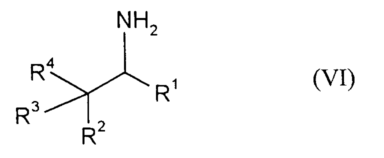
in welcher
R¹, R², R³ und R⁴ die oben angegebenen Bedeutungen haben,
in inerten Lösemitteln, in Anwesenheit von Triethylorthoformiat umsetzt.

Als Lösemittel für die einzelnen Schritte der Verfahren eignen sich die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfrakionen, oder Halogenkohlenwasserstoffe wie Dichlormethan, Trichlormethan, Tetrachlormethan, Dichlorethylen, Trichlorethylen oder Chlorbenzol, oder Essigester, Dimethylformamid, Hexamethylphosphorsäuretriamid, Acetonitril, Aceton oder Dimethoxyethan. Ebenso ist es möglich, Gemische der genannten Lösemittel zu verwenden. Besonders bevorzugt ist Acetonitril.

Das erfindungsgemäße Verfahren wird im allgemeinen in einem Temperaturbereich von 0°C bis +180°C, bevorzugt von +30°C bis +150°C durchgeführt.

Diese erfindungsgemäßen Verfahrenschritte werden im allgemeinen bei Normaldruck durchgeführt. Es ist aber auch möglich, bei Überdruck oder bei Unterdruck zu arbeiten (z.B. in einem Bereich von 0,5 bis 5 bar).

Die Verbindung der Formel (V) ist bekannt [vgl. Logemann, G. Shaw, Chemistry and Industry, 1980 (13), 541-542].

Die Amine der allgemeinen Formel (VI) sind teilweise bekannt oder neu und können dann nach bekannten Methoden hergestellt werden [vgl. L.R. Krepski et al., Synthesis, 1986, 301-303].

Die erfindungsgemäßen Verbindungen der allgemeinen Formel (I) zeigen ein nicht vorhersehbares, wertvolles pharmakologisches Wirkspektrum.

Sie inhibieren entweder eine oder mehrere der cGMP metabolisierenden Phosphodiesterasen (PDE I, PDE II und PDE V). Dies führt zu einem differenzierten Anstieg von cGMP. Eine Erhöhung des cGMP-Spiegels kann zu einer antithrombotischen, vasodilatorischen und/oder antiarrhythmischen Wirkung führen. Die Selektivität wird von der Verteilung der Isoenzyme im Gewebe mitbestimmt.

Außerdem verstärken die erfindungsgemäßen Verbindungen die Wirkung von Substanzen, wie beispielsweise EDRF (Endothelium-derived relaxing factor) und ANP (atrial natriuretic peptide), die den cGMP-Spiegel steigern.

Sie können daher in Arzneimitteln zur Behandlung von entzündlichen Erkrankungen wie beispielsweise Asthma, entzündlichen Dermatosen, zur Behandlung des Bluthochdrucks, stabiler und instabiler Angina, peripheren und kardialen Gefäßerkrankungen, von Arrhythmien, zur Behandlung von thromboembolischen Erkrankungen und Ischämien wie Myokardinfarkt, Hirnschlag, transitorische und ischämische Attacken, Angina pectoris, periphere Durchblutungsstörungen, Verhinderung von Restenosen wie nach Thrombolysetherapien, percutan transluminalen Angioplastien (PTA) und Bypass, percutan transluminalen Koronarangioplastien (PTCA), Bypass, septischem Schock und Krankheiten des Urogenitalsystems wie beispielsweise Prostatahypertrophy, Impotenz und Inkontinenz eingesetzt werden.

### Aktivität der Phosphodiesterasen (PDE's)

Die cGMP stimulierbare PDE II, die c-GMP hemmbare PDE III und die cAMP spezifische PDE IV wurden entweder aus Schweine- oder Rinderherzmyokard isoliert. Die Ca-Cal-modulinstimulierbare PDE I wurde aus Schweineaorta oder Schweinegehirn isoliert. Die cGMP spezifische PDE V wurde aus Schweinedünndarm, Schweineaorta und/oder humanen Blutplättchen gewonnen. Die Reinigung erfolgte durch Anionenaustauschchromatographie an MonoQ^{R} Pharmacia im wesentlichen nach der Methode von M. Hoey and Miles D. Houslay, Biochemical Pharmacology, Vol. 40, 193-202 (1990).

Die Bestimmung der Enzymaktivität erfolgt in einem Testansatz von 100 µl in 20 mM Tris/HCl-Puffer pH 7,5 der 5 mM MgCl₂, 0,1 mg/ml Rinderserumalbumin und entweder 800 Bq ³HcAMP oder ³HcGMP enthält. Die Endkonzentration der entsprechenden Nucleotide ist 10⁻⁶ mol/l. Die Reaktion wird durch Zugabe des Enzyms gestartet, die Enzymmenge ist so bemessen, daß während der Inkubationszeit von 30 min ca 50% des Substrates umgesetzt werden. Um die cGMP stimulierbare PDE II zu testen, wird als Substrat ³HcAMP verwendet und dem Ansatz 10⁻⁶ Mol/l nicht markiertes cGMP zugesetzt. Um die Ca-Calmodulinabhängige PDE I zu testen, werden dem Reaktionsansatz noch CaCl₂ 1 µM und Calmodulin 0,1 µM zugesetzt. Die Reaktion wird durch Zugabe von 100 µl Acetonitril, das 1 mM cAMP und 1 mM AMP enthält, gestoppt. 100 µl des Reaktionsansatzes werden auf der HPLC-Säule getrennt und die Spaltprodukte "Online" mit einem Durchflußscintillationszähler quantitativ bestimmt. Es wird die Substanzkonzentration gemessen, bei der die Reaktionsgeschwindigkeit um 50% vermindert ist.

### Inhibition der Phosphodiesterasen in vitro

| Bsp.-Nr. | PDE I IC₅₀ [µM] | PDE II IC₅₀ [µM] | PDE V IC₅₀ [µM] |
|---|---|---|---|
| 5 | 10 | 3 | 50 |
| 8 | 40 | 2 | |
| 14 | 4 | 0,6 | 0,3 |
| 20 | 1 | 0,4 | 1 |
| 25 | 3 | 0,1 | 10 |

Die Verbindungen wurden auf antihypertensive Aktivität am narkotisierten Schwein untersucht.

Die antihypertensive Aktivität wurde nach intravenöser Applikation an SHR-Ratten gemessen.

Zur Bestimmung der cyclischen Nucleotide wurden Herz- und Aortengewebe entnommen und unmittelbar tiefgefroren. Die Proben wurden unter flüssigen N₂ pulverisiert, mit 70% Ethanol extrahiert und der Gehalt am cGMP und cAMP mit kommerziellen Radioimmunoassays (Amersham) bestimmt.

Die erektionsauslösende Wirkung wurde am narkotisierten Kaninchen gemessen (C.G. Stief et al. World Journal Urology 1990, S. 233-236).

Die Substanzen wurden in Dosierungen 0,1 bis 10 mg/kg entweder direkt in den Corpus cavernosum, intraduodenal, rektal, oral, transdermal oder intravenös appliziert.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, parenteral, transdermal, perlingual oder intravenös.

Im allgemeinen hat es sich als vorteilhaft erwiesen, bei intravenöser Applikation Mengen von etwa 0,01 bis 10 mg/kg, vorzugsweise etwa 0,1 bis 10 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Ausgangsverbindungen

### Allgemeine Arbeitsvorschrift zur Synthese der 1-substituierten 5-Acylamino-imidazol-4-carboxamide (Formel IV)

### Methode A:

10 mmol des 1-substituierten 5-Amino-imidazol-4-carboxamids und 15 mmol (60%ige Dispersion in Mineralöl) NaH (bzw. 30 mmol NaH, falls R² für eine Hydroxygruppe steht) werden in 50 ml abs. THF 3 h bei 20°C gerührt (schwerlösliche Imidazole werden bis zu 3 h refluxiert). Bei 20°C werden 10 mmol Säurechlorid (bzw. 20 mmol bei Vorhandensein einer Hydroxygruppe) in 2,5 ml abs. THF zugetropft und über Nacht bei Raumtemperatur gerührt. Das Lösemittel wird im Vakuum abrotiert, der Rückstand in 50 ml Essigsäureethylester aufgenommen und mit 50 ml Wasser ausgeschüttelt. Die organische Phase wird abgetrennt, mit Na₂SO₄ getrocknet und das Lösemittel im Vakuum eingedampft. Der Rückstand wird durch Umkristallisation oder Flashchromatographie gereinigt.

### Methode B:

10 mmol des 1-substituierten 5-Amino-imidazol-4-carboxamids werden in 20 ml trockenem Pyridin gelöst. Nach Zugabe von 50 mg 4-Dimethylaminopyridin (DMAP) werden 11 mmol Säurechlorid (bzw. 22 mmol falls R² für eine Hydroxygruppe steht) bei 20°C zugetropft (feste Säurechloride werden in wenig abs. Toluol gelöst). Es wird 1 h bei 20°C gerührt, in einigen Fällen ist noch 1 - 2 stündiges Erwärmen auf 50°C notwendig (DC-Kontrolle). Der Ansatz wird in 100 ml Eiswasser gegossen und 3 mal mit je 50 ml Essigsäureethylester ausgeschüttelt. Die vereinigten Essigsäureethylesterphasen werden 2 mal mit 1 n HCl, 1 mal mit gesättigter NaCl-Lösung gewaschen, über Na₂SO₄ getrocknet und im Vakuum eingedampft. Der Rückstand wird durch Flashchromatographie oder Umkristallisation gereinigt.

Nach diesen beiden Verfahren werden die in der Tabelle I aufgeführten 1-substituierten 5-Acylamino-imidazol-4-carboxamide hergestellt:

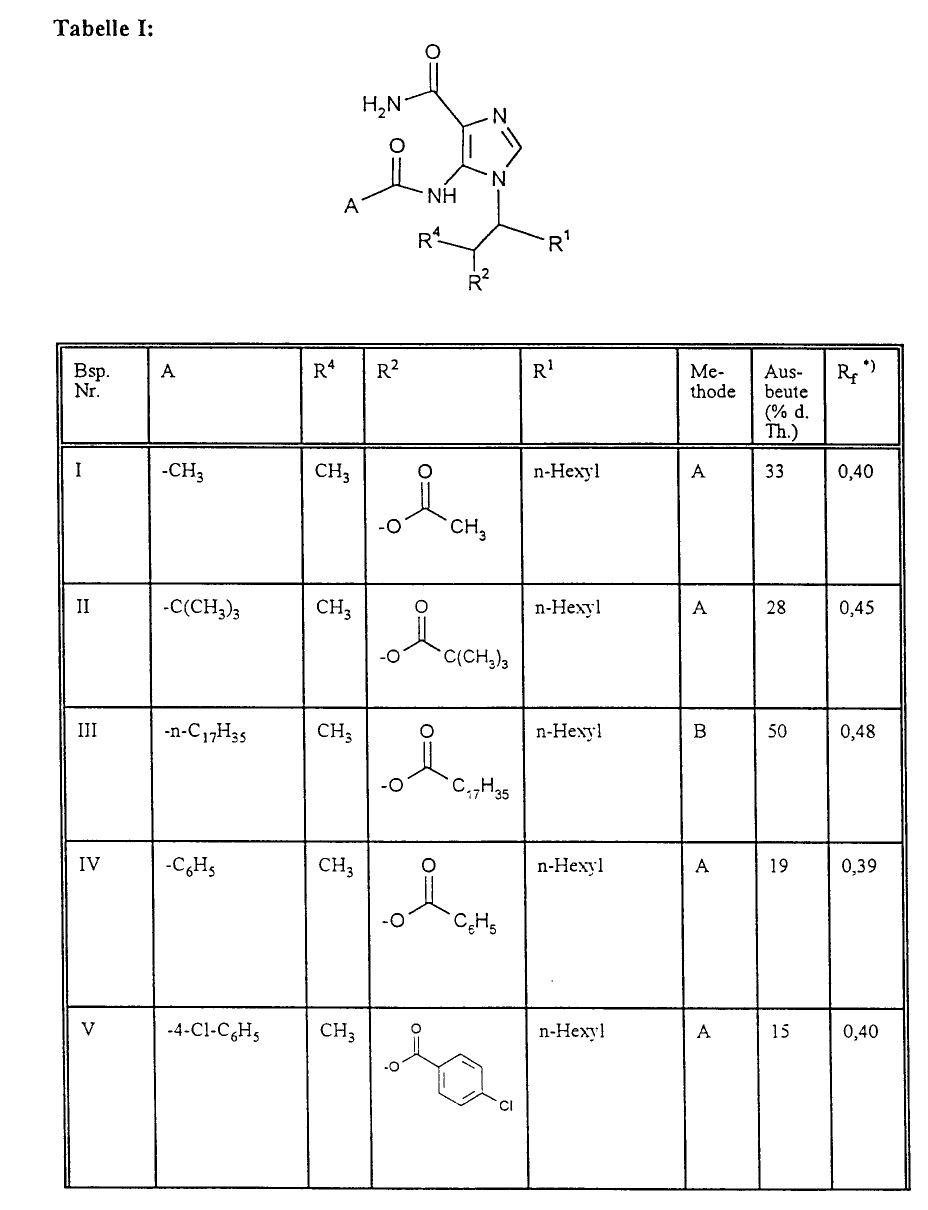

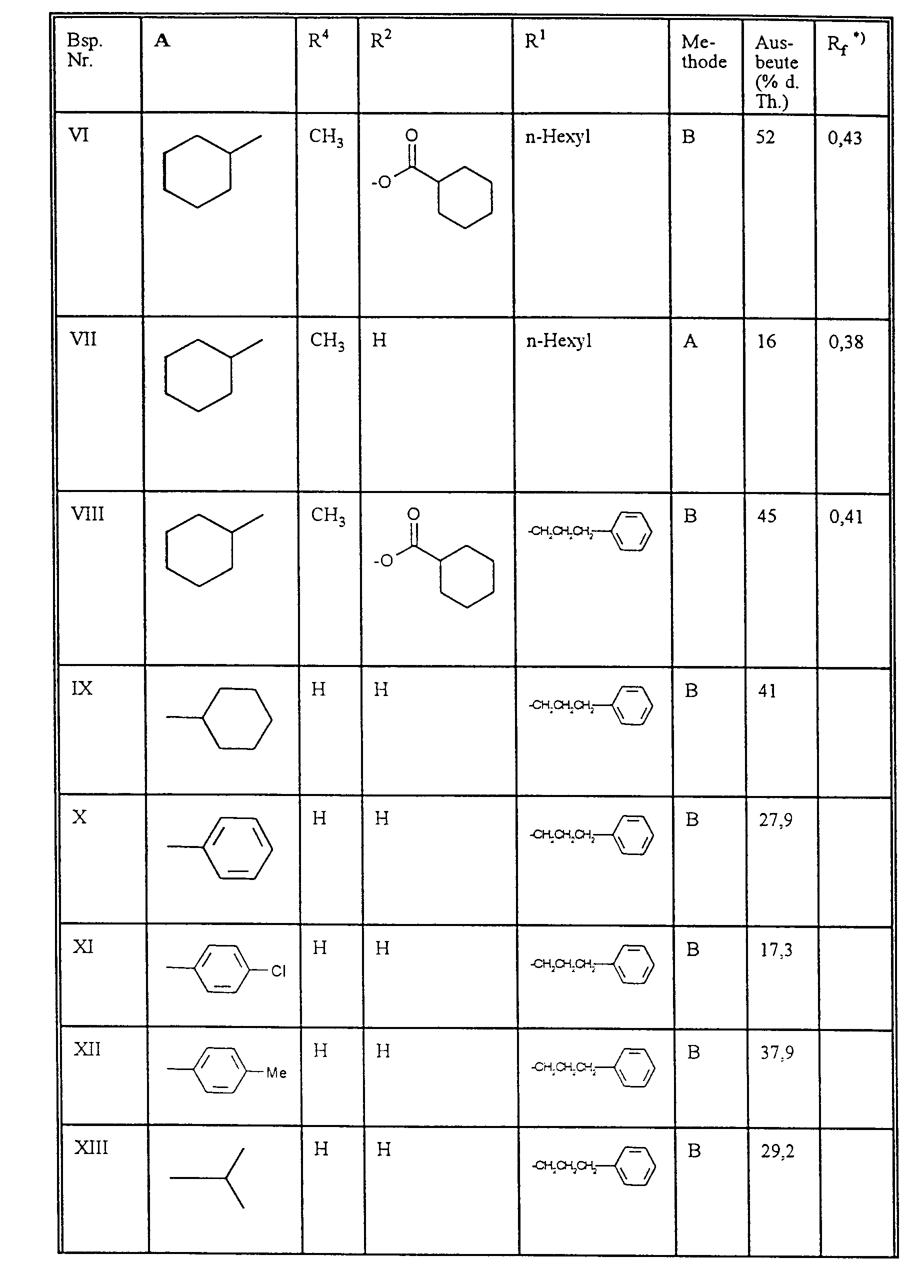

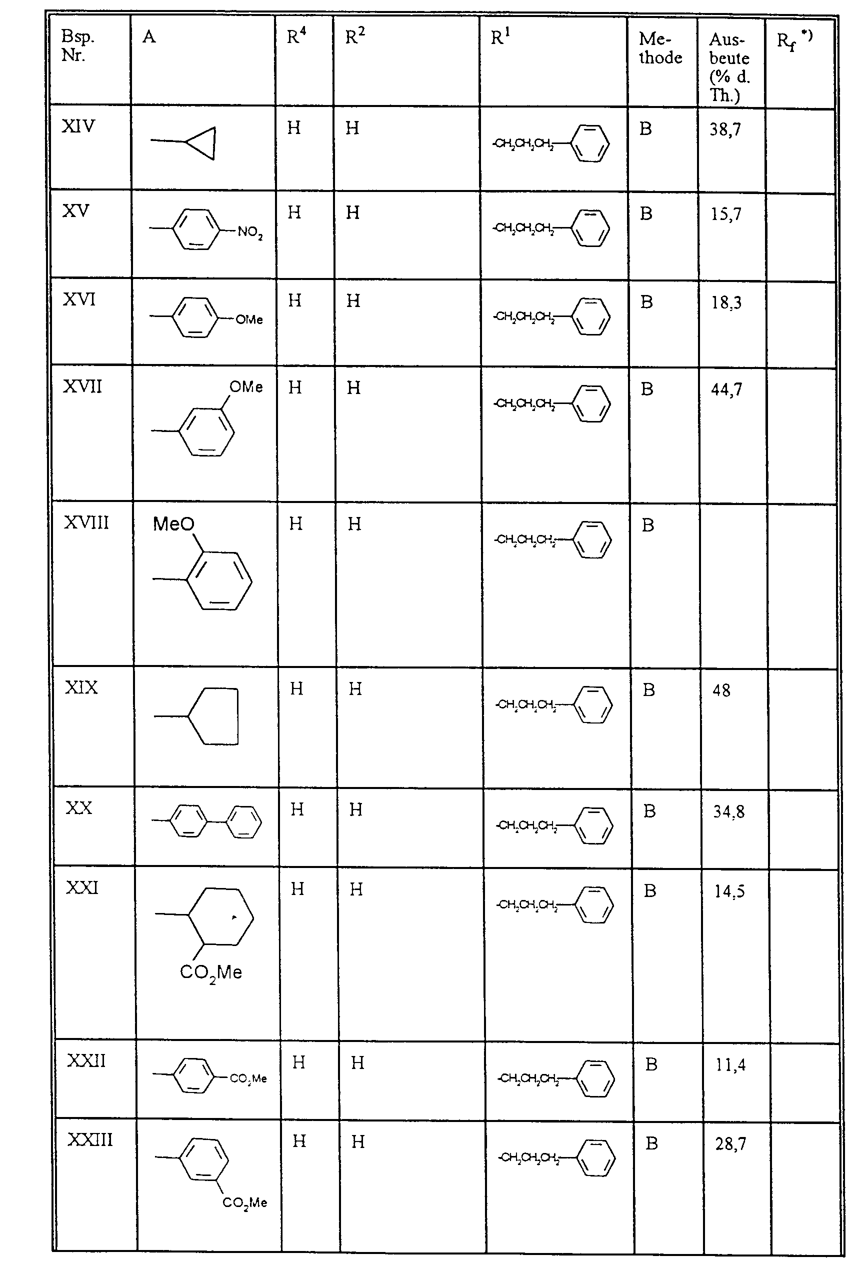

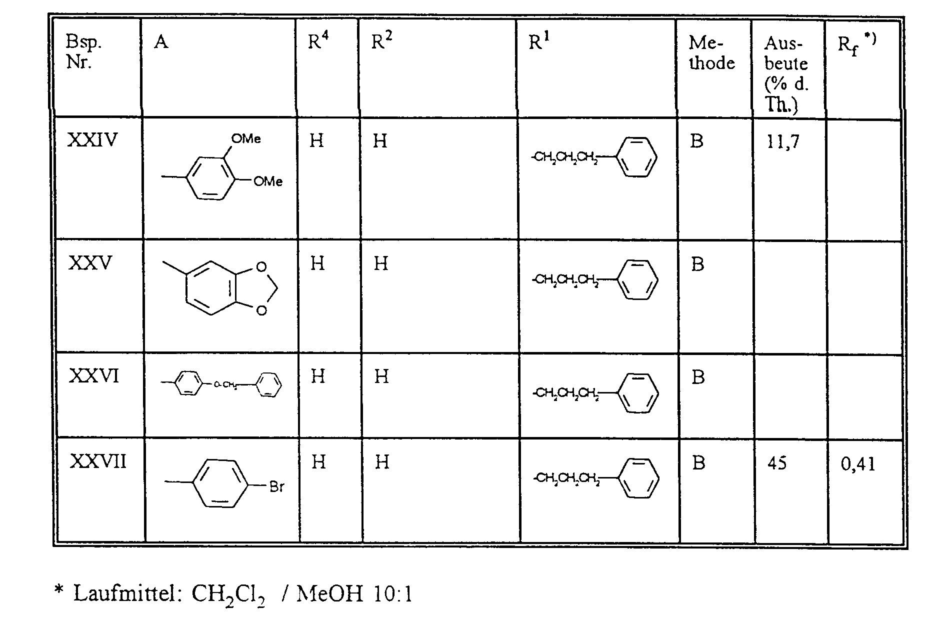

### Herstellungsbeispiele

### Allgemeine Arbeitsvorschrift zur Synthese der 2,9-disubstituierten Purin-6-one (Formel I)

### Methode A:

1 mmol 1-substituiertes 5-Acylamino-imidazol-4-carboxamid und 5 mmol Kaliumcarbonat werden in 20 ml Ethanol und 10 ml Wasser über Nacht am Rückfluß gekocht. Das Lösemittel wird im Vakuum eingedampft, der Rückstand in 20 ml Essigsäureethylester aufgenommen und mit ges. NaCl-Lösung ausgeschüttelt. Die organische Phase wird abgetrennt, über Na₂SO₄ getrocknet und im Vakuum eingedampft. Der Rückstand wird durch Umkristallisation oder Flashchromatographie gereinigt.

### Methode B: (analog EP 0526 004)

1 mmol 1-substituiertes 5-Acylamino-imidazol-4-carboxamid, 5 mmol Kaliumcarbonat und 1 ml 30% H₂O₂-Lösung werden in 10 ml Wasser und 10 ml Ethanol über Nacht am Rückfluß gekocht (DC-Kontrolle). Die weitere Aufarbeitung erfolgt in Analogie zur Vorschrift A.

Nach diesen beiden Vorschriften werden die in der Tabelle 1 aufgeführten 2,9-disubstituierten Purin-6-one hergestellt:

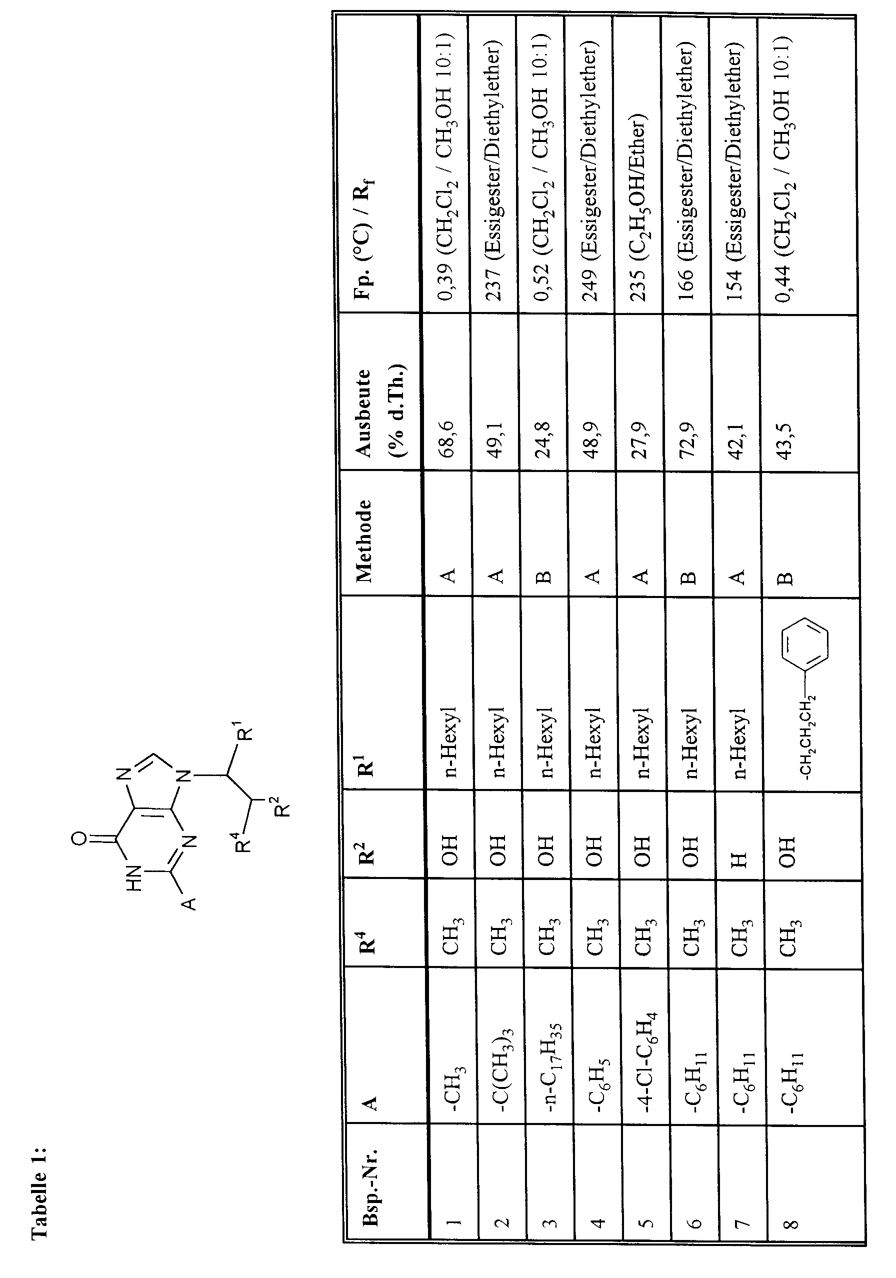

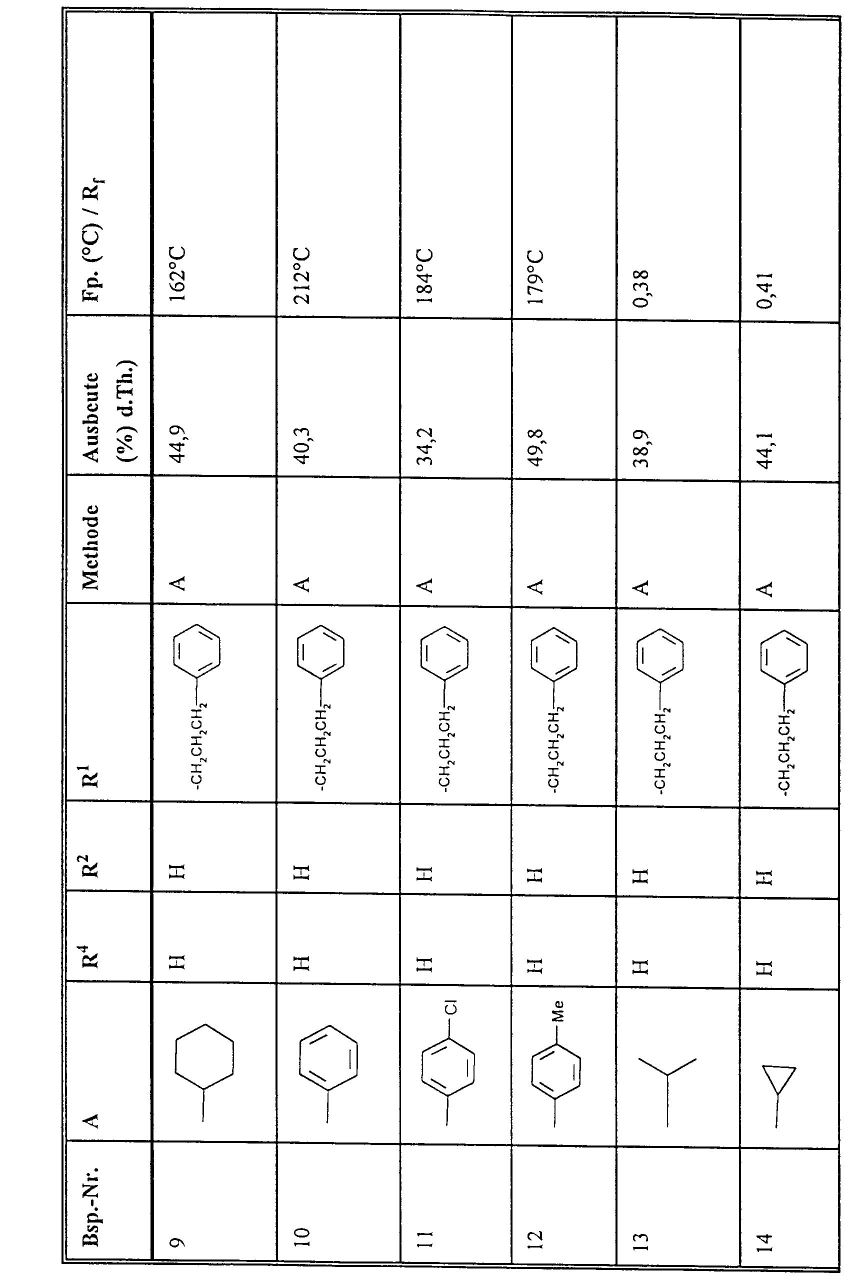

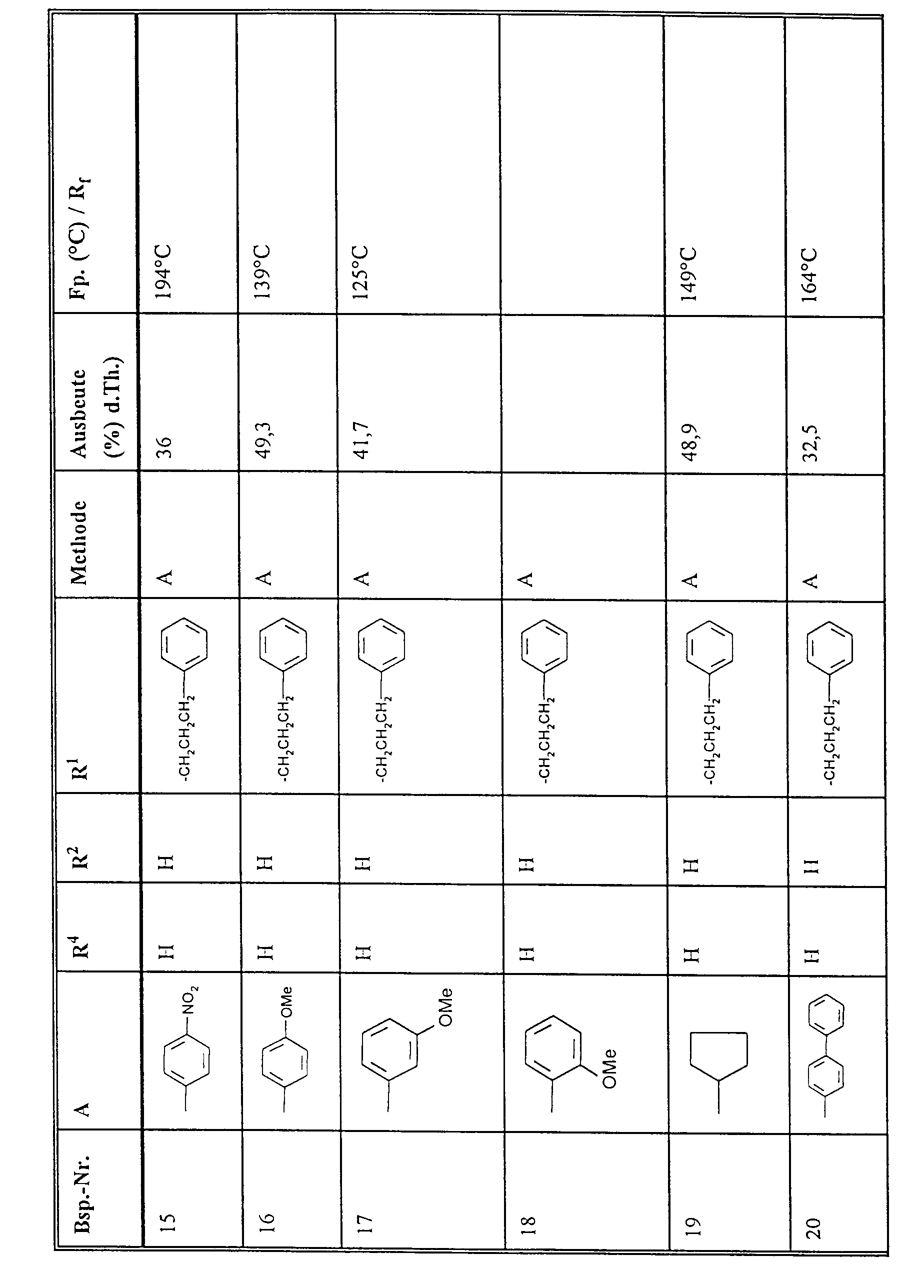

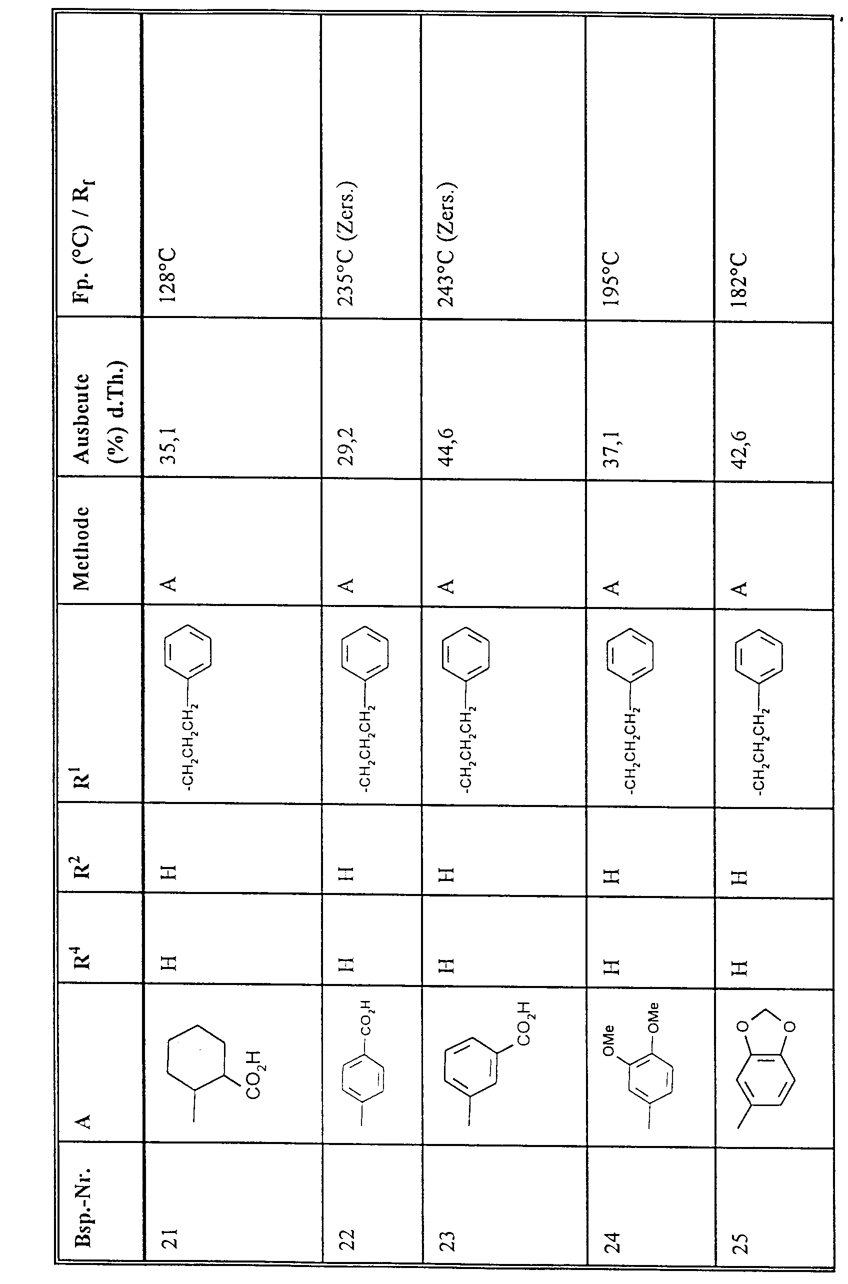

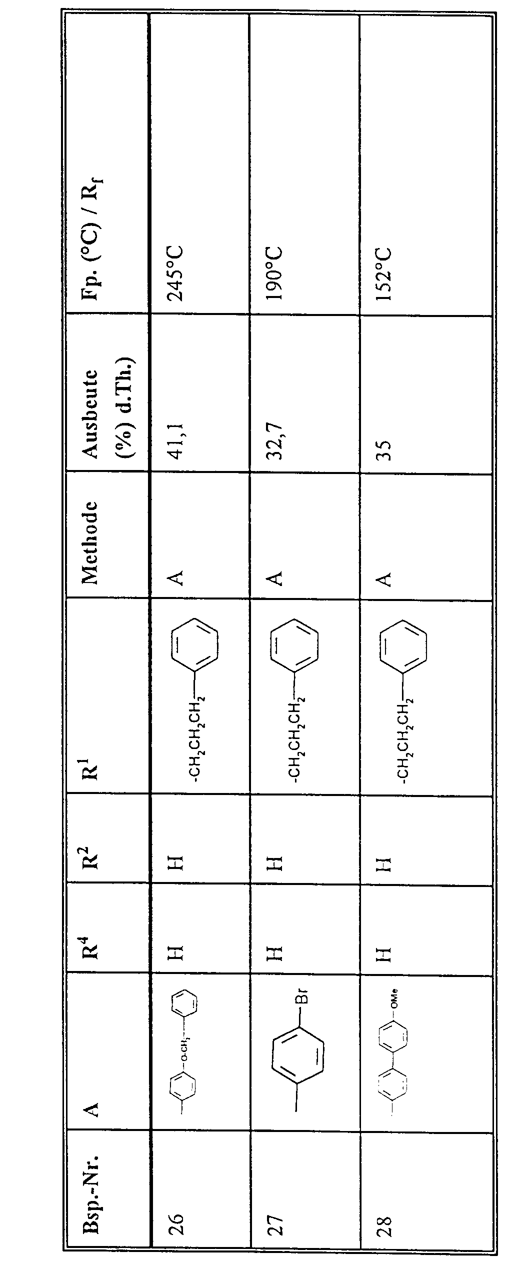

### Beispiel 29

### 9-(2-Methansulfonyloxy-3-nonyl)-2-cyclohexyl-purin-6-on

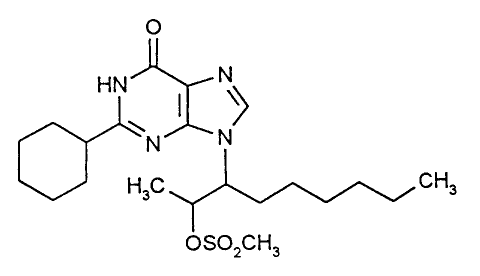

0,36 g (1 mmol) 9-(2-Hydroxy-3-nonyl)-2-cyclohexyl-purin-6-on (Beispiel 6) und 0,3 ml Triethylamin werden in 5 ml abs. CH₂Cl₂ bei 0°C gerührt. Dann werden 0,1 ml Methansulfonsäurechlorid, gelöst in 2,5 ml abs. CH₂Cl₂, zugetropft. Nach 30 min. bei 0°C wird mit 10 ml gesättigter NaHCO₃-Lösung, 10 ml 2 n HCl-Lösung und mit 10 ml gesättiger NaHCO₃-Lösung ausgeschüttelt. Die organische Phase wird über Na₂SO₄ getrocknet, das Lösemittel im Vakuum eingedampft und der Rückstand durch Flashchromatographie mit Essigsäureethylester / CH₂Cl₂ / CH₃OH 10:1 als Eluens gereinigt.
Ausbeute: 0,198 g (45,2%)
R_{f} = 0,52 (CH₂Cl₂ / CH₃OH 10:1)

### Beispiel 30

### 9-(2-Methansulfonyl-6-phenyl-3-hexyl)-2-cyclohexyl-purin-6-on

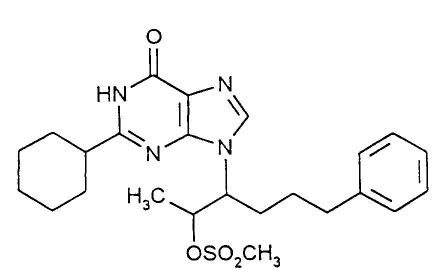

Die Titelverbindung wird in Analogie zur Vorschrift des Beispiels 29 ausgehend von 9-(2-Hydroxy-6-phenyl-3-hexyl)-2-cyclohexyl-purin-6-on (Beispiel 8) hergestellt.
Ausbeute: 89,7%
R_{f} = 0,5 (CH₂Cl₂ / CH₃OH 10:1)

### Beispiel 31

### 9-(2-Azido-3-nonyl)-2-cylcohexyl-purin-6-on

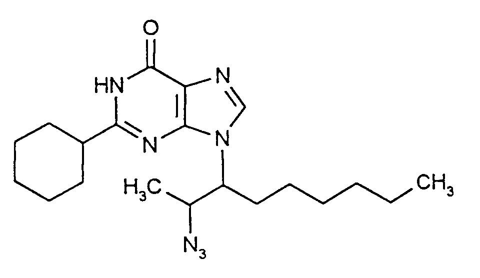

0,381 g (0,87 mmol) 9-(2-Methansulfonyloxy-3-nonyl)-2-cyclohexyl-purin-6-on (Beispiel 29) und 0,113 g (1,74 mmol) Natriumazid werden über Nacht in 5 ml abs. DMF bei 100°C gerührt. Es wird auf 20°C abgekühlt, mit 30 ml Essigsäureethylester versetzt und 2 mal mit je 50 ml Wasser und 1 mal mit 50 ml gesättigter NaCl-Lösung gewaschen. Nach Trocknen über Na₂SO₄ wird das Lösemittel im Vakuum eingedampft, und der Rückstand durch Flashchromatographie (Eluens: CH₂Cl₂ / CH₃OH 30:1) gereinigt.
R_{f} = 0,55 (CH₂Cl₂ / CH₃OH 10:1)
Ausbeute: 0,311 g (92,8%)

### Beispiel 32

### 9-(2-Azido-6-phenyl-3-hexyl)-2-cyclohexyl-purin-6-on

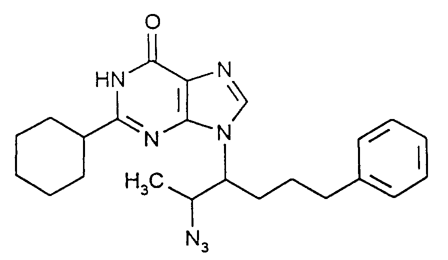

Die Titelverbindung wird in Analogie zur Vorschrift des Beispiels 31 ausgehend von 9-(2-Methansulfonyloxy-6-phenyl-3-hexyl)-2-cyclohexyl-purin-6-on (Beispiel 30) hergestellt.
Ausbeute: 0,372 g (78,2%)
R_{f} = 0,54 (CH₂Cl₂ / CH₃OH 10:1)

### Beispiel 33

### 9-(2-Oxo-3-nonyl)-2-cyclohexyl-purin-6-on

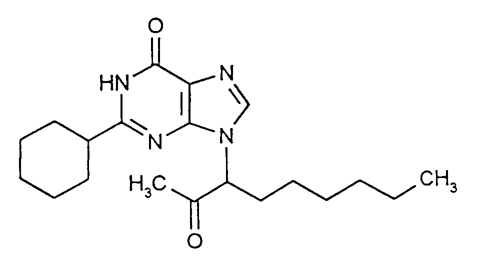

Zu 0,15 ml Oxalylchlorid in 5 ml abs. CH₂Cl₂ werden bei -60°C 0,2 ml abs. DMSO in 3 ml abs. CH₂Cl₂ zugetropft und 20 min bei -60°C nachgerührt. Dann werden langsam 540 mg (1,5 mmol) 9-(2-Hydroxy-3-nonyl)-2-cyclohexyl-purin-6-on (Beispiel 6) in 3 ml CH₂Cl₂ zugetropft und 1 h bei -60°C nachgerührt. Zu dieser Lösung werden 1 ml Triethylamin in 3 ml CH₂Cl₂ zugetropft. Man läßt auf Raumtemperatur kommen, gibt 7 ml Wasser zu und trennt die organische Phase ab. Die organische Phase wird mit 10 ml 2 n HCl und 10 ml gesättigter NaCl-Lösung gewaschen, über Na₂SO₄ getrocknet und im Vakuum eingedampft. Der Rückstand wird durch Flashchromatographie (Eluens: CH₂Cl₂ / CH₃OH 40:1) gereinigt.
Ausbeute: 0,416 g (77,4%)
R_{f} = 0,47 (CH₂Cl₂ / CH₃OH 10:1)

### Beispiel 34

### 9-(2-Oxo-6-phenyl-3-hexyl)-2-cyclohexyl-purin-6-on

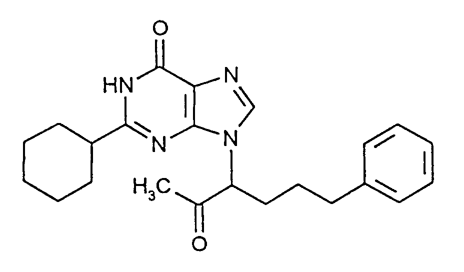

Die Titelverbindung wird in Analogie zur Vorschrift des Beispiels 33 ausgehend von 9-(2-Hydroxy-6-phenyl-3-nonyl)-2-cyclohexyl-purin-6-on (Beispiel 8) hergestellt.
R_{f} = 0,48 (CH₂Cl₂ / CH₃OH 10:1)
Ausbeute: 48,8%

## Patentansprüche

1. 2,9-Disubstituierte Purin-6-one der allgemeinen Formel (I) 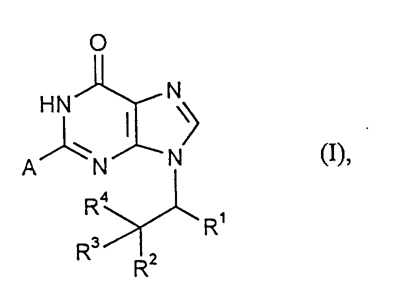 in welcher
R¹ für geradkettiges oder verzweigtes Alkyl mit 2 bis 10 Kohlenstoffatomen steht, das gegebenenfalls durch Phenyl substituiert ist, das seinerseits durch Halogen, Nitro, Cyano oder durch geradkettiges oder verzweigtes Alkyl mit bis zu 6 Kohlenstoffatomen substituiert sein kann,
R² für Wasserstoff, Hydroxy, Azido oder für geradkettiges oder verzweigtes Alkyl mit bis zu 6 Kohlenstoffatomen steht, oder für eine Gruppe der Formel -O-SO₂R⁵ steht,
worin
R⁵ geradkettiges oder verzweigtes Alkyl mit bis zu 4 Kohlenstoffatomen oder Phenyl bedeutet,
R³ für Wasserstoff steht,
oder
R² und R³ gemeinsam den Rest der Formel =O bilden,
R⁴ für Wasserstoff oder für geradkettiges oder verzweigtes Alkyl mit bis zu 4 Kohlenstoffatomen steht,
und
A für einen Rest der Formel 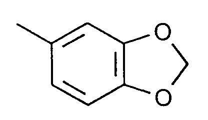 oder
für geradkettiges oder verzweigtes Alkyl mit bis zu 20 Kohlenstoffatomen steht, oder
für Cycloalkyl mit 3 bis 7 Kohlenstoffatomen oder
für Phenyl steht, die gegebenenfalls bis zu 2-fach gleich oder verschieden durch Halogen, Trifluormethyl, Carboxyl, Nitro, Cyano oder durch geradkettiges oder verzweigtes Alkyl, Alkoxycarbonyl oder Alkoxy mit jeweils bis zu 5 Kohlenstoffatomen substituiert sind, die ihrerseits durch Phenyl substituiert sein können, und/oder die Cyclen gegebenenfalls durch Phenyl substituiert sind, das seinerseits durch geradkettiges oder verzweigtes Alkoxy mit bis zu 5 Kohlenstoffatomen substituiert sein kann,
und deren Tautomere und Salze.

2. 2,9-Disubstituierte Purin-6-one der Formel nach Anspruch 1,
in welcher
R¹ für geradkettiges oder verzweigtes Alkyl mit 2 bis 8 Kohlenstoffatomen steht, das gegebenenfalls durch Phenyl substituiert ist, das seinerseits durch Fluor, Chlor, Brom, Nitro, Cyano oder durch geradkettiges oder verzweigtes Alkyl mit bis zu 4 Kohlenstoffatomen substituiert sein kann,
R² für Wasserstoff, Hydroxy, Azido oder für geradkettiges oder verzweigtes Alkyl mit bis zu 4 Kohlenstoffatomen oder eine Gruppe der Formel
-O-SO₂-R⁵ steht,
worin
R⁵ geradkettiges oder verzweigtes Alkyl mit bis zu 3 Kohlenstoffatomen oder Phenyl bedeutet,
R³ für Wasserstoff steht,
oder
R² und R³ gemeinsam den Rest der Formel =O bilden,
R⁴ für Wasserstoff oder für geradkettiges oder verzweigtes Alkyl mit bis zu 3 Kohlenstoffatomen steht, und
A für einen Rest der Formel 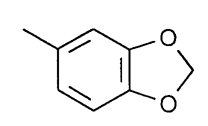 oder
für geradkettiges oder verzweigtes Alkyl mit bis zu 19 Kohlenstoffatomen steht, oder
für Cyclopropyl, Cyclopentyl, Cyclohexyl, Cylcoheptyl oder
für Phenyl steht, die gegebenenfalls bis zu 2-fach gleich oder verschieden durch Fluor, Chlor, Brom, Nitro, Carboxyl, Hydroxy oder durch geradkettiges oder verzweigtes Alkyl, Alkoxycarbonyl oder Alkoxy mit jeweils bis zu 4 Kohlenstoffatomen substituiert sind, die ihrerseits durch Phenyl substituiert sein können, und/oder die Cyclen gegebenenfalls durch Phenyl substituiert sind, das seinerseits durch geradkettiges oder verzweigtes Alkoxy mit bis zu 4 Kohlenstoffatomen substituiert ist,
und deren Tautomere und Salze.

3. 2,9-Disubstituierte Purin-6-one der Formel nach Anspruch 1
in welcher
R¹ für geradkettiges oder verzweigtes Alkyl mit 2 bis 7 Kohlenstoffatomen steht, das gegebenenfalls durch Phenyl substituiert ist, das seinerseits durch Fluor, Chlor, Brom, Nitro, Cyano, durch geradkettiges oder verzweigtes Alkyl mit bis zu 3 Kohlenstoffatomen substituiert sein kann,
R² für Wasserstoff, Hydroxy, Azido oder für geradkettiges oder verzweigtes Alkyl mit bis zu 3 Kohlenstoffatomen oder eine Gruppe der Formel
- OSO₂R⁵ steht,
worin
R⁵ geradkettiges oder verzweigtes Alkyl mit bis zu 3 Kohlenstoffatomen oder Phenyl bedeutet,
R³ für Wasserstoff steht,
oder
R² und R³ gemeinsam den Rest der Formel =O bilden,
R⁴ für Wasserstoff oder für geradkettiges oder verzweigtes Alkyl mit bis zu 3 Kohlenstoffatomen steht, und
A für einen Rest der Formel 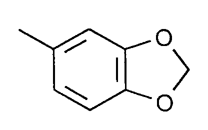 oder
für geradkettiges oder verzweigtes Alkyl mit bis zu 18 Kohlenstoffatomen steht, oder
für Cyclopropyl, Cyclopentyl, Cyclohexyl, Cycloheptyl oder
für Phenyl steht, die gegebenenfalls bis zu 2-fach gleich oder verschieden durch Fluor, Chlor, Brom, Hydroxy, Carboxyl, Nitro oder durch geradkettiges oder verzweigtes Alkyl, Alkoxycarbonyl oder Alkoxy mit jeweils bis zu 3 Kohlenstoffatomen substituiert sind, die ihrerseits durch Phenyl substituiert sein können, und/oder die Cyclen gegebenenfalls durch Phenyl substituiert sind, das seinerseits durch geradkettiges oder verzweigtes Alkoxy mit bis zu 3 Kohlenstoffatomen substituiert sein kann,
und deren Tautomere und Salze.

4. 2,9-Disubstituierte Purin-6-one nach Anspruch 1 bis 3 zur therapeutischen Anwendung.

5. Verfahren zur Herstellung von 2,9-disubstituierten Purin-6-onen nach Anspruch 1 bis 3, dadurch gekennzeichnet, daß man zunächst Verbindungen der allgemeinen Formel (II) 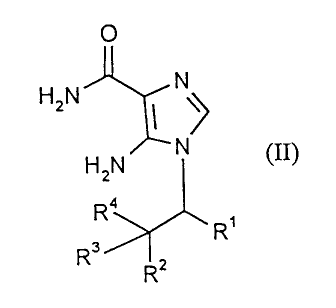 in welcher
R¹, R², R³ und R⁴ die oben angegebenen Bedeutungen haben,
durch Umsetzung mit Verbindungen der allgemeinen Formel (III)
A-CO-Cl (III)
in welcher
A die oben angegebene Bedeutung hat,
in inerten Lösemitteln und in Anwesenheit einer Base,
in die Verbindungen der allgemeinen Formel (IV) 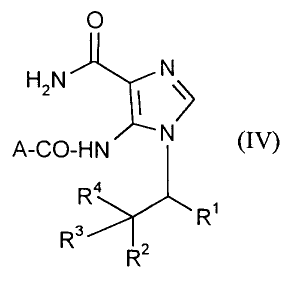 in welcher
A, R¹, R², R³ und R⁴ die oben angegebene Bedeutung haben,
überführt,
in einem zweiten Schritt in inerten Lösemitteln und in Anwesenheit einer Base cyclisiert,
und die Substituenten R¹, R², R³ und R⁴ durch Acylierung, Oxidation und/oder Azidaustausch einführt bzw. derivatisiert.

6. Arzneimittel enthaltend 2,9-disubstituierte Purin-6-one nach Anspruch 1 bis 3.

7. Arzneimittel nach Anspruch 6 zur Behandlung von Entzündungen, thromboembolischen Erkrankungen sowie Herz- und Kreislauferkrankungen.

8. Verwendung von 2,9-disubstituierten Purin-6-onen nach Anspruch 1 bis 3 zur Herstellung von Arzneimitteln.

9. Verwendung nach Anspruch 8 zur Herstellung von Arzneimitteln zur Behandlungen von Entzündungen, thromboembolischen Erkrankungen sowie Herz- und Kreislauferkrankungen.

10. Arzneimitteln nach Anspruch 6 bis 7 zur Behandlung der Impotenz.
